# EUROPEAN PATENT SPECIFICATION

(11) **EP 3 776 583 B1**
(45) Date of publication and mention of the grant of the patent: **08.06.2022**
(21) Application number: 19715460.2
(22) Date of filing: 02.04.2019
(51) Int. Cl.: G16H 40/67

(54) **GUIDANCE METHOD AND SYSTEM FOR TELEDENTISTRY IMAGING**
FÜHRUNGSVERFAHREN UND -SYSTEM FÜR DIE BILDGEBUNG IN DER ZAHNMEDIZIN
PROCÉDÉ ET SYSTÈME DE GUIDAGE POUR IMAGERIE DE TÉLÉDENTISTERIE

(30) Priority: 02.04.2018 US 201862651373 P
(43) Date of publication of application: 17.02.2021
(73) Proprietor: Koninklijke Philips N.V., 5656 AG Eindhoven (NL)
(72) Inventor: BEVIS, Taylor, 5656 AE Eindhoven (NL); JEANNE, Vincent, 5656 AE Eindhoven (NL)
(74) Representative: Philips Intellectual Property & Standards
(86) International application number: PCT/EP2019/058244
(87) International publication number: WO 2019/192987

(56) References cited:
- US-A1- 2007 171 220
- US-A1- 2007 172 112
- US-A1- 2014 272 765

## Description

### FIELD OF THE DISCLOSURE

The present disclosure is directed generally to methods and systems for obtaining high-quality oral images for teledentistry.

### BACKGROUND

Individuals are generally instructed to undergo oral checkups at least twice a year to ensure proper oral care, and many dental insurance programs provide coverage for these oral checkups. Between checkups there may be situations where an individual or an oral care professional desires a review of their oral health, including an indication that the condition of their oral health has or has not adversely changed.

Teledentistry, which is the transfer of dentistry information such as clinical information and/or oral images over a telecommunications system, offers a system for oral review or analysis between in-person checkups. Teledentistry is a rapidly growing service as it offers a low-cost and low-burden solution utilizing technology, namely smartphones or other cameras, to which individuals readily have access. On-demand dental care has the potential to provide consumers with greater peace of mind and expedited treatment when an intervention from a dental professional is required. Furthermore, teledentistry can lower the cost of dental services, and can improve access to and delivery of dental services. Indeed, in some situations, teledentistry may be a replacement for an in-person checkup.

Teledentistry necessarily requires dental images obtained by an individual, typically obtained by the individual without assistance. The individual cannot visualize the screen while the imaging device, such as a smartphone, is in position to collect the content requested by the dental professional. This often leads to many trial and error shots to capture sufficiently accurate and high-quality images of the target teeth. The utility of an image for assessing oral health is determined by many features, including whether the image properly contains the target teeth, the clarity of the image, the lighting within the environment, and/or the viewing angle, among others. An image may be determined to be of high-quality when the details in the image facilitate understanding of the patient's oral health by a professional.

Invariably, some or all of the images obtained by the individual and sent to the dental professional are unusable due to failure to capture target teeth or regions of the mouth, poor clarity, poor lighting, improper angles, and/or many other reasons. It is common, therefore, for dental professionals to either use inferior images in an analysis, or to request additional images from the individual. Both scenarios are inefficient, expensive, and potentially delay treatment.

US 2014/272765 A1 discloses a system and a method in which data is received from a dental imaging system. The received data is analyzed to adjust one or more imaging parameters of the dental imaging system. The imaging parameters include exposure of the image. The system establishes whether the exposure parameter lies within a dynamic range of the imaging system. The system adjusts the exposure parameter iteratively in real time.

Accordingly, there is a continued need in the art for teledentistry systems and methods that ensure the capture of high-quality oral images.

### SUMMARY OF THE DISCLOSURE

The present disclosure is directed to inventive methods and systems for obtaining high-quality images. Various embodiments and implementations herein are directed to an imaging system configured to identify high-quality images. The imaging system comprises an imager configured to obtain one or more images of the mouth. A processor extracts one or more features from the images, including features related to image quality and features related to object recognition. The processor analyzes the extracted features to determine whether they satisfy a predetermined feature threshold. If the extracted features do not satisfy the predetermined feature threshold, the system uses the extracted features and a determined orientation of the imaging device to calculate a physical adjustment required to satisfy the predetermined feature threshold. This required physical adjustment is provided to the user, and a new image is obtained after the physical adjustment is implemented. Sufficiently high-quality images can be transmitted to a dental professional where they can be utilized for analysis by the professional.

Generally in one aspect, a method for obtaining one or more images using an imaging device is provided. The method includes: (i) obtaining, using an imager of the imaging device, an image; (ii) determining, by a sensor of the imaging device, an orientation of the imaging device; (iii) extracting, by a controller of the imaging device, one or more features from the obtained image, wherein the extracted one or more features comprises at least an image quality feature and an object recognition feature; (iv) determining, by the controller, whether the one or more extracted features satisfy a predetermined feature threshold; (v) calculating, by the controller if the one or more extracted features does not satisfy the predetermined feature threshold, a physical adjustment of the imaging device required to satisfy the predetermined feature threshold, wherein the calculation of the required physical adjustment is based on the one or more extracted features and the determined orientation of the imaging device; and (vi) providing, via a user interface of the imaging device, feedback to a user regarding the obtained image if the one or more extracted features does not satisfy the predetermined feature threshold, wherein the feedback comprises instructions to implement the calculated physical adjustment of the imaging device required to satisfy the predetermined feature threshold.

According to an embodiment, the method further includes: obtaining, using the imager of the imaging device, an updated image after a physical adjustment is implemented; extracting, by the controller, one or more features from the updated image, wherein the extracted one or more features comprises at least an image quality feature and an object recognition feature; determining, by the controller, that the one or more extracted features satisfy the predetermined feature threshold; and transmitting the updated image to a dental professional.

According to an embodiment, the method further includes storing the updated image in memory.

According to an embodiment, the method further includes updating the orientation of the imaging device if the imaging device detects a movement above a predetermined threshold.

According to an embodiment, the feedback comprises audio, visual, and/or haptic feedback.

According to an embodiment, the imaging device is a smartphone and the imager is a smartphone camera.

According to an embodiment, the one or more imaging sensor parameters are controlled or determined by image properties at one or more points within an obtained image. According to an embodiment, the image quality feature comprises blur quantification, specular highlight quantification, and/or dynamic range detection.

According to an aspect, an imaging device for obtaining one or more images is provided. The device includes: an imager configured to obtain one or more images; a user interface configured to provide feedback to a user of the imaging device; a communications module configured to transmit one or more images; an orientation sensor configured to determine an orientation of the imaging device; and a controller configured to: (i) extract one or more features from the obtained image, wherein the extracted one or more features comprises at least an image quality feature and an object recognition feature; (ii) determine whether the one or more extracted features satisfy a predetermined feature threshold; (iii) calculate, if the one or more extracted features does not satisfy the predetermined feature threshold, a physical adjustment of the imaging device required to satisfy the predetermined feature threshold, wherein the calculation of the required physical adjustment is based on the one or more extracted features and the determined orientation of the imaging device; and (iv) provide, via the user interface, feedback to the user regarding the obtained image if the one or more extracted features does not satisfy the predetermined feature threshold, wherein the feedback comprises instructions to implement the calculated physical adjustment of the imaging device required to satisfy the predetermined feature threshold.

According to an embodiment, the controller is further configured to: (i) extract one or more features from an updated image obtained after a physical adjustment is implemented by the user, wherein the extracted one or more features comprises at least an image quality feature and an object recognition feature; (ii) determine, by the decision module, that the one or more extracted features satisfy the predetermined feature threshold; and (iii) direct the communications module to transmit the updated image.

According to an aspect, an imaging system configured to obtain one or more high-quality images is provided. The system includes: an imaging device comprising: (i) an imager configured to obtain one or more images; (ii) a communications module configured to transmit one or more images; and (iii) an orientation sensor configured to determine an orientation of the imaging device. The system also includes a processing device comprising: a communications module configured to receive one or more images from the imaging device; and a controller configured to: (i) extract one or more features from the obtained image, wherein the extracted one or more features comprises at least an image quality feature and an object recognition feature; (ii) determine whether the one or more extracted features satisfy a predetermined feature threshold; (iii) calculate, if the one or more extracted features does not satisfy the predetermined feature threshold, a physical adjustment of the imaging device required to satisfy the predetermined feature threshold, wherein the calculation of the required physical adjustment is based on the one or more extracted features and the determined orientation of the imaging device; and (iv) provide, via a user interface, feedback to the user regarding the obtained image if the one or more extracted features does not satisfy the predetermined feature threshold, wherein the feedback comprises instructions to implement the calculated physical adjustment of the imaging device required to satisfy the predetermined feature threshold.

As used herein for purposes of the present disclosure, the term "controller" is used generally to describe various apparatus relating to the operation of an imaging apparatus, system, or method. A controller can be implemented in numerous ways (e.g., such as with dedicated hardware) to perform various functions discussed herein. A "processor" is one example of a controller which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

The term "user interface" as used herein refers to an interface between a human user or operator and one or more devices that enables communication between the user and the device(s). Examples of user interfaces that may be employed in various implementations of the present disclosure include, but are not limited to, switches, potentiometers, buttons, dials, sliders, track balls, display screens, various types of graphical user interfaces (GUIs), touch screens, microphones and other types of sensors that may receive some form of human-generated stimulus and generate a signal in response thereto.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the disclosure will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the disclosure.
FIG. 1 is a schematic representation of an imaging device, in accordance with an embodiment.
FIG. 2 is a schematic representation of an imaging system, in accordance with an embodiment.
FIG. 3 is a flowchart of a method for obtaining a dental image, in accordance with an embodiment.
FIG. 4 is a schematic representation of feedback provided to a user of an imaging device or system, in accordance with an embodiment.

### DETAILED DESCRIPTION OF EMBODIMENTS

The present disclosure describes various embodiments of a method and device for teledentistry. More generally, Applicant has recognized and appreciated that it would be beneficial to provide a system to ensure that high-quality images are obtained by an individual. Accordingly, the methods described or otherwise envisioned herein provide an imaging device or system such as a smartphone, smart mirror, and/or other imaging device configured to obtain one or more images of the individual's dental region. The imaging device or system includes a processor configured to extract one or more features from the images, including features related to image quality and features related to object recognition. The processor analyzes the extracted features to determine whether they satisfy a predetermined feature threshold. If the extracted features do not satisfy the predetermined feature threshold, the system uses the extracted features and a determined orientation of the imaging device to calculate a physical adjustment required to satisfy the predetermined feature threshold. This required physical adjustment is provided to the user, and a new image is obtained after the physical adjustment is implemented. Sufficiently high-quality images can be transmitted to a dental professional where they can be utilized for analysis by the professional.

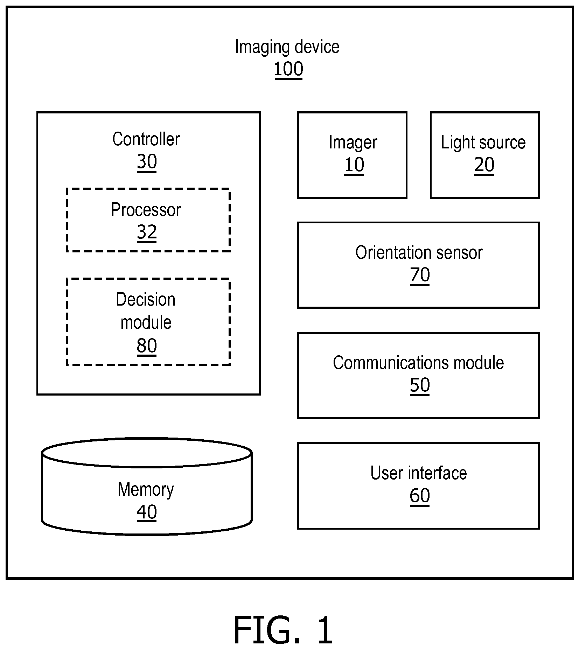

Referring to FIG. 1, in one embodiment, is an imaging device 100 configured to obtain images from a user's mouth. Imaging device 100 may be any device with an imager capable of obtain images, preferably in a digital format. For example, imaging device 100 may be a smartphone, smart mirror, wearable computing device, digital camera, laptop, and/or any other computing device or capture device capable of capturing images. The imaging device 100 may optionally comprise software such as an application which facilitates one or more aspects of the imaging system or method as described or otherwise envisioned herein.

Imaging device 100 comprises an imager 10 configured to obtain images from a user's mouth. Imager 10 is an image sensor such as a CCD or CMOS sensor, among others. For example, imager 10 may be a standalone digital camera, or may be a camera integrated into an oral care device, a smartphone, a smart mirror, a wearable device, and/or any other computing or image capture device. The imaging device 100 or imager 10 may comprise or otherwise be in communication with a light source 20 configured to illuminate one or more regions of the mouth. For example, light source 20 may be a flash or other light source associated with the device 100 or system. Light source 20 can be or comprise any light source, such as an LED light source, that emits light capable of facilitating high-quality oral imaging. According to an embodiment, the light source may comprise light from two or more light sources. The imager 10 and/or light source 20 may be configured to operate periodically, continuously, and/or in response to a stimulus. For example, the imager 10 and light source 20 can obtain an image in response to a user taking an image, or in response to a user positioning the imager over a portion of the oral cavity, as detected by the imager in real-time.

Imaging device 100 further comprises a controller 30 configured to receive the one or more images obtained from the imager 10. Controller 30 may be formed of one or multiple modules, and can configured to operate the imager 10 in response to an input, such as input obtained via a user interface. Controller 30 can comprise, for example, at least a processor 32. The processor 32 may take any suitable form, including but not limited to a microcontroller, multiple microcontrollers, circuitry, a single processor, or plural processors. Controller 30 and/or imaging device 100 may also comprise a memory 40. The memory 40 can take any suitable form, including a non-volatile memory and/or RAM. The non-volatile memory may include read only memory (ROM), a hard disk drive (HDD), or a solid state drive (SSD). The memory can store, among other things, an operating system. The RAM is used by the processor for the temporary storage of data. According to an embodiment, an operating system may contain code which, when executed by controller 30, controls operation of the hardware components of imaging device 100.

Imaging device 100 further comprises a communications module 50 configured to receive and/or transmit information via a wired and/or wireless communications network. The communications module 50 can be any module, device, or means capable of transmitting a wired or wireless signal, including but not limited to a Wi-Fi, Bluetooth, near field communication, and/or cellular module. The communications module 50 can, for example, transmit one or more images obtained by the imager.

Imaging device 100 further comprises an orientation sensor 70 configured to obtain or detect an orientation of the imaging device. Orientation sensor 70 can comprise, for example, a 6-axis or a 9-axis spatial sensor system, and can include one or more of an accelerometer, a gyroscope, and/or a magnetometer to provide readings relative to axes of motion of the device, and to characterize the orientation and displacement of the device. For example, sensor 70 can be configured to provide readings of six axes of relative motion (three axes translation and three axes rotation), using for example a 3-axis gyroscope and a 3-axis accelerometer. Many other configurations are possible. Other sensors may be utilized either alone or in conjunction with the sensor, including but not limited to a pressure sensor (e.g. Hall effect sensor) and other types of sensors, such as a sensor measuring electromagnetic waveforms on a predefined range of wavelengths, a capacitive sensor, a camera, a photocell, a visible light sensor, a near-infrared sensor, a radio wave sensor, and/or one or more other types of sensors. Many different types of sensors could be utilized, as described or otherwise envisioned herein. According to one possible embodiment, the sensor or sensors provide complementary information about the position of the device with respect to a user's body part, a fixed point, and/or one or more other positions.

According to an embodiment, imaging device 100 includes a user interface 60 configured to provide information to a user and/or receive information from a user. The user interface 60 can take many different forms, but is configured to provide information to the user and/or receive information from the user. For example, the information can be read, viewed, heard, felt, and/or otherwise interpreted. Accordingly, the user interface may be a display that provides information to the user, a haptic mechanism that provides haptic feedback to the user, a speaker to provide sounds or words to the user, a simple LED or array of LEDs, or any of a variety of other user interface mechanisms. According to an embodiment, the user interface 60 provides feedback to a user as images are obtained or after images are obtained. For example, user interface 60 can notify a user when an image is not of sufficient quality as determined by the methods described or otherwise envisioned herein. User interface 60 can also provide instructions or guidance to the user about images to obtain, or about improving images, among many other types of information and guidance as described or otherwise envisioned herein.

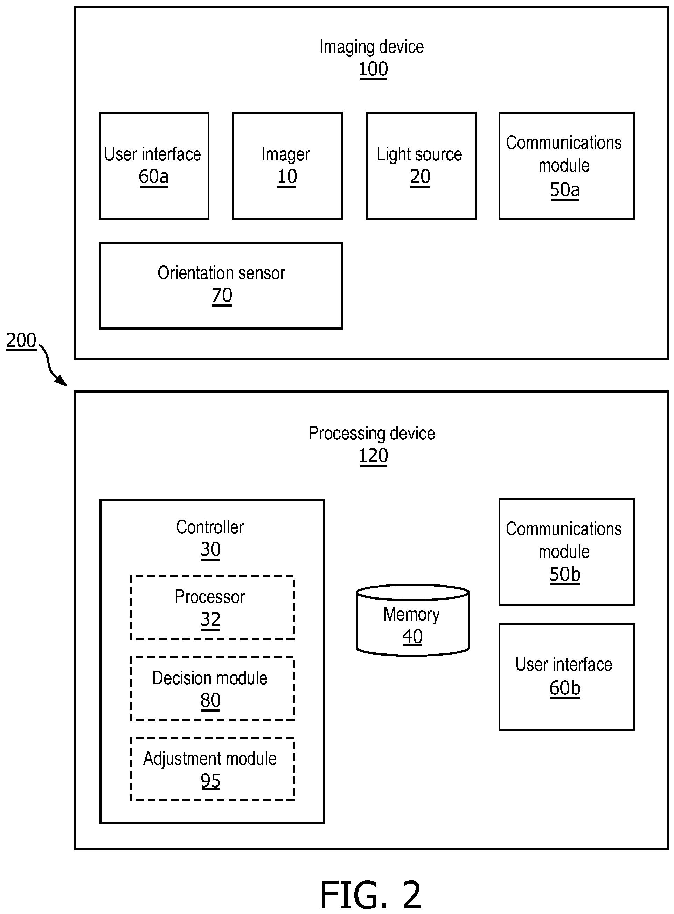

Referring to FIG. 2, in one embodiment, is an imaging system 200 configured to obtain high-quality images from a user's mouth. According to this embodiment, imaging system 200 comprises an imaging device 100 and a processing device 120. Imaging device 100 is configured to obtain one or more images of the user's mouth, and to transmit those images to the processing device 120 which may be locally or remotely located, or even be part of the imaging device 100. Processing device 120 is configured to receive and analyze the one or more images received from the imaging device, and to transmit high-quality images, where quality is determined by the methods described or otherwise envisioned herein, to a dental professional.

Imaging device 100 can be any device with an imager capable of obtain oral images, preferably in a digital format. For example, imaging device 100 may be a smartphone, smart mirror, wearable computing device, digital camera, laptop, and/or any other computing device or capture device capable of capturing images. The imaging device 100 may optionally comprise software such as an application which facilitates one or more aspects of the imaging system or method as described or otherwise envisioned herein. Imaging device 100 comprises an imager 10, such as a CCD or CMOS sensor, among others, configured to obtain images from a user's mouth. Imaging device 100 may be a standalone digital camera, or may be a camera integrated into an oral care device, a smartphone, a smart mirror, a wearable device, and/or any other computing device. Imaging device 100 may comprise a light source 20 configured to illuminate one or more regions of the mouth. The device may also comprise a user interface.

The imaging device 100 also comprises a communications module 50a configured to receive and/or transmit information via a wired and/or wireless communications network. The communications module 50a can be any module, device, or means capable of transmitting a wired or wireless signal, including but not limited to a Wi-Fi, Bluetooth, near field communication, and/or cellular module. The communications module 50a can, for example, transmit one or more images obtained by the imager to the processing device 120.

The imaging device 100 also comprises an orientation sensor 70 configured to obtain or detect an orientation of the imaging device. Orientation sensor 70 can comprise, for example, a 6-axis or a 9-axis spatial sensor system, and can include one or more of an accelerometer, a gyroscope, and/or a magnetometer to provide readings relative to axes of motion of the device, and to characterize the orientation and displacement of the device. For example, sensor 70 can be configured to provide readings of six axes of relative motion (three axes translation and three axes rotation), using for example a 3-axis gyroscope and a 3-axis accelerometer. Many other configurations are possible, and other sensors may be utilized either alone or in conjunction with orientation sensor 70 to determine orientation of the imaging device.

According to an embodiment, imaging device 100 also includes a user interface 60a, such as user interface 60 as described previously herein, configured to provide information to a user and/or receive information from a user. The user interface 60a can take many different forms, and is configured to provide information to the user and/or receive information from the user. According to an embodiment, the user interface 60a provides feedback to a user as images are obtained or after images are obtained. For example, user interface 60a can notify a user when an image is not of sufficient quality as determined by the methods described or otherwise envisioned herein. User interface 60a can also provide instructions or guidance to the user about images to obtain, or about improving imaging, among many other types of information and guidance.

Processing device 120 can be any device configured to receive images from the imaging device 100. For example, processing device 120 may be a smartphone, smart mirror, wearable computing device, laptop, and/or any other computing device. Processing device 120 may optionally comprise software such as an application which facilitates one or more aspects of the imaging system or method as described or otherwise envisioned herein.

Processing device 120 comprises a controller 30 configured to receive the one or more images obtained from the imager. Controller 30 may be formed of one or multiple modules, and can comprise, for example, processor 32. The processor 32 may take any suitable form, including but not limited to a microcontroller, multiple microcontrollers, circuitry, a single processor, or plural processors. Processing device 120 may comprise a memory 40, which can take any suitable form, including a non-volatile memory and/or RAM. The memory 40 can be configured to store one or more received images or any other information or instructions.

The processing device further comprises a communications module 50b configured to receive and/or transmit information via a wired and/or wireless communications network, including information transmitted from communications module 50a of the imaging device 100. The communications module 50b can be any module, device, or means capable of transmitting a wired or wireless signal, including but not limited to a Wi-Fi, Bluetooth, near field communication, and/or cellular module.

According to an embodiment, processing device 120 includes a user interface 60b configured to provide information to a user and/or receive information from a user. The user interface 60b can take many different forms, and is configured to provide information to the user and/or receive information from the user. According to an embodiment, the user interface 60b provides feedback to a user as images are obtained or after images are obtained. For example, user interface 60b can notify a user when an image is not of sufficient quality as determined by the methods described or otherwise envisioned herein. User interface 60b can also provide instructions or guidance to the user about images to obtain, or about improving imaging, among many other types of information and guidance.

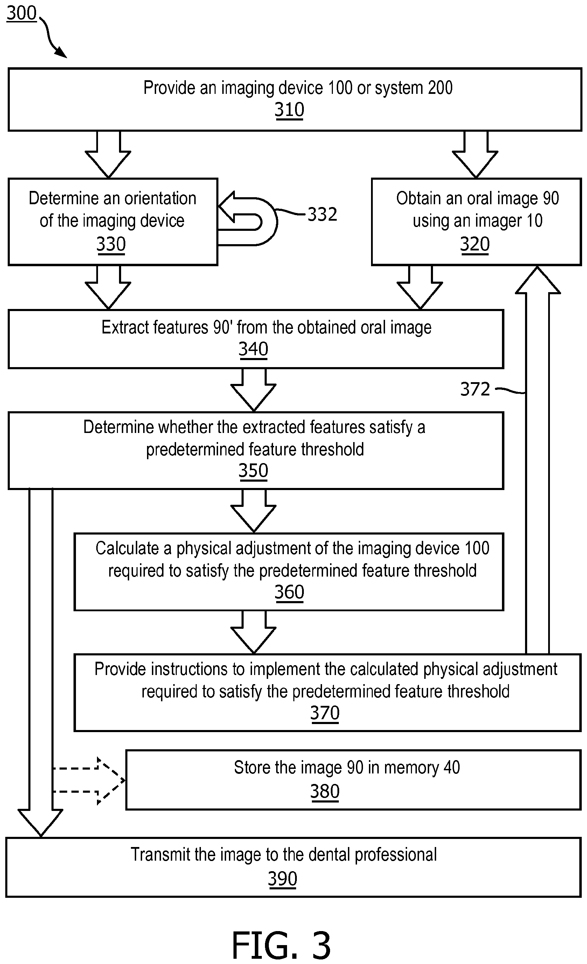

Referring to FIG. 3, in one embodiment, is a flowchart of a method 300 for obtaining high-quality images 90 from a user's mouth. At step 310, an imaging system is provided. The imaging system may be any of the imaging systems described or otherwise envisioned herein. For example, the imaging system may be imaging device 100 or imaging system 200, among many other devices or systems. Generally, the imaging system will comprise an imager 10 configured to obtain one or more images 90 of a user's mouth, a controller 30 configured to receive and analyze the obtained one or more images, a communications module 50 configured to transmit and/or receive information over a wired and/or wireless communications system, an orientation sensor 70 configured to detect an orientation of the device, and a user interface 60 to receive information from a user and/or provide information to a user. Although method 300 is described within the framework of imaging device 100 and imaging system 200, the method can be implemented using any other appropriately configured imaging device or system as described or otherwise envisioned herein.

At step 320 of the method, one or more images 90 of the user's mouth are obtained using imager 10 of the imaging device 100. According to a preferred embodiment the imager is a 2D imaging sensor integrated into a smart connected medium such as a smartphone, laptop, or other connected camera that enables the capture of wavelengths in the visible part of the spectrum. In an optional embodiment the acquisition of wavelengths can include near- to mid-infrared wavelengths using imaging sensors sensitive in the relevant parts of the light spectrum.

According to an embodiment, one or more imaging sensor parameters are controlled or determined by image properties at predetermined key points within an obtained image 90, which can be either preprogrammed or detected. For example, the camera's automatic white balance can be set based on the average properties of the color level identified on teeth areas, which can be detected either by means of an image overlay that the user manually aligns to or by detecting teeth using color properties in a heuristic or machine learning fashion. In another embodiment, the camera exposure is determined only by the pixel properties contained within a specific key area, such as the inside of the user's mouth, rather than the entire field of view of the image. This specific control ensures that the image capture is solely focused on providing the best sensor settings for the area of interest only. Thus, areas of the mouth, cheek, body, or background that are not desired are deemed irrelevant for further analysis by a dental professional.

According to an embodiment, image capture comprises capturing one or multiple images. For example, the system may obtain a series of images, such as the 'live' photo as enabled by Apple iOS, to allow either: (i) the system to automatically select the best image according to the criteria set forth in or by the decision module; or (ii) the dental professional to select the most appropriate image or images from the series for analysis.

According to an embodiment, the user may wish to obtain images 90 to share with a dental professional, either in response to injury, pain, or other stimulus, or as part of a scheduled checkup or other routine maintenance or care. The user may receive a notification or other stimulus to obtain the one or more images. Imaging device 100 may comprise software such as an app that provides a notification to a user that images are required. For example, imaging device 100 may be a smartphone with an app installed that provides notifications to obtain routine images. Alternatively, the user may instigate a teledentistry session by accessing software or an online website or service and will be prompted to obtain one or more images. Accordingly, imaging device 100 may receive instructions about which images to obtain for the session, and these instructions may be prompted, in whole or in part, based on information from the user and/or the dental professional. For example, the user may be prompted to obtain images at a specific angle, or to include certain teeth in the field of view, among many other possible prompts.

The images obtained by the user via the imager may be of any portion of the user's mouth, including but not limited to gums, teeth, tongue, or any other part of the oral cavity. The images may be analyzed immediately or may be stored for batch processing in real-time or near real-time. The images may be retained by the device for analysis, or may be transmitted to another device for downstream analysis as described or otherwise envisioned herein. The images 90 are transmitted to and/or received by controller 30 for feature extraction and analysis.

At step 330 of the method, which can be performed at any point during the method, the orientation sensor 70 of imaging device 100 obtains sensor data regarding an orientation of the device. The orientation sensor, such as an accelerometer, obtains data continuously or periodically. The orientation sensor uses the information to determine an orientation of the device, or communicates the sensor data to a controller or other processor to determine an orientation of the device. According to an embodiment, the orientation sensor is a component of the controller of the imaging device. The controller may continuously or periodically receive sensor data, or may receive sensor data in response to a query or request. The imaging device can use any method or system to analyze the sensor data to determine an orientation of the device. For example, the imaging device may determine that is it substantially horizontal with a certain calculated angle relative to horizontal, or it may determine that it is vertical with a certain calculated tilt relative to vertical. In this way, the imaging device can detect its orientation and monitor its movement over time.

The orientation sensor 70 can continuously or periodically monitor the orientation and/or movement of the imaging device. This may facilitate the feedback and/or physical adjustment provided to the user in downstream steps of the method, as the user may move the device between images or between taking an image and receiving feedback about the image. Accordingly, at step 332 of the method, the orientation sensor 70 and/or the controller obtains or requests new orientation data. In this way, the device can monitor the orientation and/or movement of the imaging device over time.

According to an embodiment, the imaging device may obtain or request new orientation data if the imaging device or orientation sensor detects a movement above a predetermined threshold. For example, the imaging device or orientation sensor may be configured to monitor for movement after taking an image, and may trigger a request for updated orientation data if movement is detected, or if movement above a threshold is detected. Since significant movement after an image is taken can result in a new position or orientation of the device, information about the new position or orientation will be necessary for downstream steps of the method.

At step 340 of the method, controller 30 extracts one or more image quality features 90'from the obtained one or more oral images 90. According to an embodiment, controller 30 extracts at least two sets of features from the one or more images, including at an image quality feature and an object recognition feature, although many other feature types are possible.

According to an embodiment, controller 30 extracts one or more image quality features 90', which targets characterization of the impact a user has on the image acquisition process. The image quality feature 90'can include, for example, blur quantification using discrete wavelet transform or frequency analysis to quantify the amount of high frequency components available in the region of interest within an image. As another example, the image quality feature may include specular highlight quantification using either detection of 'hotspots' using thresholding and/or a Gaussian filter. As another example, the image quality feature may include dynamic range detection by computing the available distribution of color in each channel over the region of interest. The image quality feature 90' may include other image quality features in addition to those described herein.

According to an embodiment, controller 30 extracts an image content feature, which targets characterization of the impact the image sensor has on the image acquisition process. The image content feature can include, for example, filtering of the output using a dedicated template. Since each image is expected to represent a specific portion of the mouth related to a diagnostic process, correlating the captured image with a predetermined template will provide a metric characterizing how alike the captured image and targets are. The process may utilize Haar-like features, color-based features, or other features. As another example, the image content feature can comprise, utilize, or by analyzed by a trained detector. Since each image is expected to contain a specific portion of the mouth such as teeth or gums, trained detectors which can optional utilize machine learning algorithms can provide a metric characterizing the presence or absence of such key mouth parts essential to the analysis performed by the dental professional. The image quality feature may include other image content features in addition to those described herein.

According to an embodiment, the features can be extracted or analyzed using any feature extraction method, algorithm, or filter. For example, the feature extraction method, algorithm, or filter may comprise color detection, Canny edge detection, Deriche edge detection, a Sobel operator or filter, Harris corner detection, Gaussian filters, blob detection based on the Laplacian of the Gaussian, a Hessian matrix, local binary patterns, and/or any other feature extraction method, algorithm, or filter.

Once extracted, the one or more features 90' can be analyzed in real-time or near real-time, or can be stored for subsequent analysis. For example, the extracted one or more features can be stored in memory 40, and extracted features 90' can be associated in memory with the image 90 or images from which they were obtained.

At step 350 of the method, the imaging system determines whether the one or more extracted features 90' satisfy a predetermined feature threshold. According to an embodiment, the imaging device or system comprises a Decision Module 80 which is configured to analyze the one or more extracted features 90'. Decision Module 80 effectively gates the transmission of image data to the dental professional. If the image quality is poor and/or doesn't contain the requested features, feedback will be provided to the user. If the image quality is sufficiently high and/or contains the requested features, the image can be transmitted to the dental professional.

Decision Module 80 may be programmed or configured, for example, to compare the one or more extracted features 90' to a predetermined feature threshold to determine whether the image is of sufficient quality to deem it a high-quality image that may be transmitted to and/or utilized by a dental professional. An image 90 will either be accepted or rejected based on the comparison. According to an embodiment, Decision Module 80 is programmed or configured to analyze the one or more extracted features 90' using a heuristic model or function. According to another embodiment, Decision Module 80 is programmed or configured to analyze the one or more extracted features using a classification system such as a decision tree, a naive Bayes classifier, a support vector machine, or similar classifier or algorithm. According to another embodiment, Decision Module 80 is programmed or configured to analyze the one or more extracted features using machine learning and/or artificial intelligence such as neural networks and/or ensemble methods.

At step 360 of the method, if the extracted features 90' do not satisfy the predetermined feature threshold, the controller calculates a physical adjustment of the imaging device required to satisfy the predetermined feature threshold, based on the extracted features and the determined orientation of the imaging device. According to an embodiment, the imaging device or system comprises an Adjustment Module 95 which is configured to analyze the one or more extracted features and/or the determined orientation of the imaging device to determine what physical adjustment would potentially correct any issues detected in the obtained image. According to an embodiment, the required position change is calculated using the result from Decision Module 80 to determine the actual position and view of mouth geometry. According to an embodiment, the transform from a current view to a target view could be accomplished, for example, via a mesh model or other method or mechanism, and the transform could be applied to the device orientation to determine required movement (e.g. tilt upward/downward, rotate left/right, etc.).

For example, the Adjustment Module 95 may determine that moving the camera slightly to the left would bring the target area properly into the field of view. The Adjustment Module 95 may determine that moving the camera slightly away from the target area would bring the target area properly into focus. The Adjustment Module 95 may determine that tilting the camera would bring the target area properly into the field of view. Many other physical adjustments, movements, or other modifications are possible.

At step 370 of the method, the device provides feedback to the user regarding the obtained oral image 90. The feedback includes instructions to implement the calculated physical adjustment of the imaging device required to satisfy the predetermined feature threshold. The feedback may include instructions to move the camera slight to the left or right from the current position, to move the camera closer to or away from the target, to tilt the camera in a direction, to rotate the camera in a direction, and/or any other adjustment.

The feedback can be provided via the user interface 60, or via any other mechanism. For example, the user can receive a visual, haptic, audible, or other notification that the image is insufficient. Audio feedback may comprise recorded commands to move or tilt, beeps, tones, or other methods to indicate to the user how to move the device in order to achieve the target image. Visual feedback may comprise an LED or screen flash where the flash rate and/or intensity changes as the user approaches the target image, for example. Haptic feedback may include a change in vibration pattern and/or intensity to indicate when the user is approaching the target image. A connected secondary device can display feedback and/or live-view comparing current view to the target to supplement instructions. For example, processing device 120 may comprise a screen or other user interface 60b that provides feedback to the user.

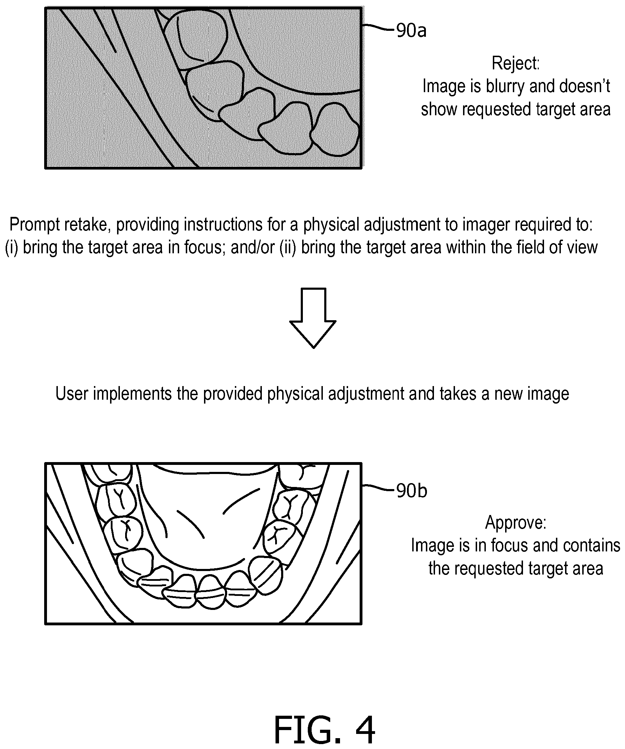

Referring to FIG. 4, for example, in the top panel the image 90a obtained by the user is blurry and doesn't contain the proper portion of the mouth. Accordingly, at step 350 of the method, the Decision Module 80 determines that the one or more extracted features 90' do not satisfy the predetermined feature threshold. At step 360 of the method, the system calculates a physical adjustment of the imaging device required to satisfy the predetermined feature threshold, and at step 370 of the method the device provides feedback to the user comprising the calculated physical adjustment. The user takes a new image 90b which, as a result of the implemented physical adjustment, is no longer blurry and contains the target area.

At step 372 of the method, the user implements the feedback and makes the necessary physical adjustment, or attempts to make the necessary physical adjustment, and returns to step 320 to obtain 320 to obtain an additional one or more images using the imager. The method then proceeds through steps 330 through 350 to determine whether the new images satisfy the predetermined feature threshold, and thus whether the user's physical adjustment remedied any deficiency detected in the original images. These and other steps of the method can be repeated an unlimited number of times.

If Decision Module 80 determines that the extracted features satisfy the predetermined threshold, either with an original image or with a new image obtained after a physical adjustment provided via feedback in step 370, the image 90 is ready for storage and/or transmission. Accordingly, the imaging system can then authorize or initiate transmission of the obtained oral image to a dental professional. This prevents wasted time or inferior diagnosis due to poor images, which is an ongoing issue in current embodiments of teledentistry.

At optional step 380 of the method, the imaging device saves the obtained image in temporary or permanent storage. The image can be stored before or after transmission to the dental professional. The storage can be any storage or memory, such as memory 40 on the imaging device, in the processing device, on the cloud, or in any remote or local storage device, server, or database.

At step 390 of the method, images 90 determined to be of sufficiently high quality and/or containing the desired or requested portion of the mouth can be transmitted to the dental professional or a service utilized by the dental professional in real-time as images are authorized, or only after a predetermined number or quality of images are obtained. Images can be transmitted to the dental professional or other third-party system or service via wired and/or wireless communication using a communications module 50 of the imaging device or system. The system may be configured or designed to only transmit images to a dental professional or other third-party system or service if a requested number of images of sufficiently high quality are obtained, and/or if certain regions are captured in images of sufficiently high quality. For example, the system may be configured to require images of sufficiently high quality of both the top and bottom teeth, or of front and back teeth, or of a certain tooth, before it will authorize transmission of the images to the dental professional or other third-party system or service.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

## Claims

1. A method (300) for obtaining one or more oral images using an imaging device (100), comprising:
obtaining (320), using an imager (10) of the imaging device, an oral image (90);
determining (330), by a sensor (70) of the imaging device, an orientation of the imaging device (100);
extracting (340), by a controller (30) of the imaging device, one or more features (90') from the obtained oral image, wherein the extracted one or more features comprises at least an image quality feature and an object recognition feature;
determining (350), by the controller, whether the one or more extracted features satisfy a predetermined feature threshold;
calculating (360), by the controller if the one or more extracted features does not satisfy the predetermined feature threshold, a physical adjustment of the imaging device required to satisfy the predetermined feature threshold, wherein the calculation of the required physical adjustment is based on the one or more extracted features and the determined orientation of the imaging device; and
providing (370), via a user interface (60) of the imaging device, feedback to a user regarding the obtained oral image if the one or more extracted features does not satisfy the predetermined feature threshold, wherein the feedback comprises instructions to implement the calculated physical adjustment of the imaging device required to satisfy the predetermined feature threshold.

2. The method of claim 1, further comprising the steps of:
obtaining (320), using the imager of the imaging device, an updated oral image after a physical adjustment is implemented;
extracting (340), by the controller, one or more features from the updated oral image, wherein the extracted one or more features comprises at least an image quality feature and an object recognition feature;
determining (350), by the controller, that the one or more extracted features satisfy the predetermined feature threshold; and
transmitting (390) the updated oral image to a dental professional.

3. The method of claim 2, further comprising the step of storing (380) the updated oral image in memory.

4. The method of claim 1, further comprising updating (332) the orientation of the imaging device if the imaging device detects a movement above a predetermined threshold.

5. The method of claim 1, wherein the feedback comprises audio, visual, and/or haptic feedback.

6. The method of claim 1, wherein the imaging device is a smartphone and the imager is a smartphone camera.

7. The method of claim 1, wherein one or more imaging sensor parameters are controlled or determined by image properties at one or more points within an obtained oral image.

8. The method of claim 1, wherein the image quality feature comprises blur quantification, specular highlight quantification, and/or dynamic range detection.

9. An imaging device (100) for obtaining one or more oral images, comprising:
an imager (10) configured to obtain one or more images (90);
a user interface (60) configured to provide feedback to a user of the imaging device;
a communications module (50) configured to transmit one or more oral images;
an orientation sensor (70) configured to determine an orientation of the imaging device; and
a controller (30) configured to: (i) extract one or more features from the obtained oral image, wherein the extracted one or more features comprises at least an image quality feature and an object recognition feature; (ii) determine whether the one or more extracted features satisfy a predetermined feature threshold; (iii) calculate, if the one or more extracted features does not satisfy the predetermined feature threshold, a physical adjustment of the imaging device required to satisfy the predetermined feature threshold, wherein the calculation of the required physical adjustment is based on the one or more extracted features and the determined orientation of the imaging device; and (iv) provide, via the user interface, feedback to the user regarding the obtained oral image if the one or more extracted features does not satisfy the predetermined feature threshold, wherein the feedback comprises instructions to implement the calculated physical adjustment of the imaging device required to satisfy the predetermined feature threshold.

10. The imaging device of claim 9, wherein the controller is further configured to: (i) extract one or more features from an updated oral image obtained after a physical adjustment is implemented by the user, wherein the extracted one or more features comprises at least an image quality feature and an object recognition feature; (ii) determine, by the decision module, that the one or more extracted features satisfy the predetermined feature threshold; and (iii) direct the communications module to transmit the updated oral image.

11. The imaging device of claim 10, wherein the controller is further configured to store the updated oral image in memory.

12. The imaging device of claim 10, wherein the feedback comprises audio, visual, and/or haptic feedback.

13. The imaging device of claim 10, wherein the imaging device is a smartphone and the imager is a smartphone camera.

14. An imaging system (200) configured to obtain one or more high-quality oral images, comprising:
an imaging device (100) comprising an imager (10) configured to obtain one or more oral images (90); a communications module (50b) configured to transmit one or more oral images; and an orientation sensor (70) configured to determine an orientation of the imaging device; and
a processing device (120) comprising: a communications module (50b) configured to receive one or more oral images from the imaging device; and a controller (30) configured to: (i) extract one or more features (90') from the obtained oral image, wherein the extracted one or more features comprises at least an image quality feature and an object recognition feature; (ii) determine whether the one or more extracted features satisfy a predetermined feature threshold; (iii) calculate, if the one or more extracted features does not satisfy the predetermined feature threshold, a physical adjustment of the imaging device required to satisfy the predetermined feature threshold, wherein the calculation of the required physical adjustment is based on the one or more extracted features and the determined orientation of the imaging device; and (iv) provide, via a user interface (60a, 60b), feedback to the user regarding the obtained oral image if the one or more extracted features does not satisfy the predetermined feature threshold, wherein the feedback comprises instructions to implement the calculated physical adjustment of the imaging device required to satisfy the predetermined feature threshold.

15. The imaging system of claim 14, wherein the feedback comprises audio, visual, and/or haptic feedback.

## Patentansprüche

1. Eine Methode (300) zum Erhalten einer oder mehrerer oraler Bilder unter Verwendung eines Bildgebungsgeräts (100), umfassend:
Erhalten (320), unter Verwendung eines Bilderzeugers (10) des Bildgebungsgeräts, eines oralen Bildes (90);
Bestimmung (330), durch einen Sensor (70) des Bildgebungsgeräts, einer Ausrichtung des Bildgebungsgeräts (100);
Extrahieren (340) eines oder mehrerer Merkmale (90') aus dem erhaltenen oralen Bild durch eine Steuerung (30) des Bildgebungsgeräts, wobei das eine oder die mehreren extrahierten Merkmale mindestens ein Bildqualitätsmerkmal und ein Objekterkennungsmerkmal umfassen; Bestimmen (350) durch die Steuerung, ob das eine oder die mehreren extrahierten Merkmale einen vorbestimmten Merkmalsschwellenwert erfüllen;
Berechnen (360), durch die Steuerung, ob das eine oder die mehreren extrahierten Merkmale den vorbestimmten Merkmalsschwellenwert nicht erfüllen, ob eine physikalische Anpassung des Bildgebungsgeräts erforderlich ist, um den vorbestimmten Merkmalsschwellenwert zu erfüllen, wobei die Berechnung der erforderlichen physikalischen Anpassung auf dem einen oder den mehreren extrahierten Merkmalen und der ermittelte Ausrichtung des Bildgebungsgeräts beruht; und
Bereitstellen (370), über eine Benutzerschnittstelle (60) des Bildgebungsgeräts, einer Rückmeldung an einen Benutzer in Bezug auf das erhaltene orale Bild, wenn das eine oder die mehreren extrahierten Merkmale den vorbestimmten Merkmalsschwellenwert nicht erfüllen, wobei die Rückmeldung Anweisungen für die berechnete physikalische Anpassung des Bildgebungsgeräts umfasst, die erforderlich ist, um den vorbestimmten Merkmals-Schwellenwert zu erfüllen.

2. Methode nach Anspruch 1, ferner umfassend die Schritte: Erhalten (320) eines aktualisierten oralen Bildes unter Verwendung des Bildgebers des Bildgebungsgeräts, nachdem eine physikalische Anpassung vorgenommen wurde; Extrahieren (340) eines oder mehrerer Merkmale aus dem aktualisierten oralen Bild durch die Steuerung, wobei das eine oder die mehreren extrahierten Merkmale mindestens ein Bildqualitätsmerkmal und ein Objekterkennungsmerkmal umfassen;
Bestimmen (350), durch das Steuergerät, dass das eine oder die mehreren extrahierten Merkmale den vorbestimmten Merkmals-Schwellenwert erfüllen; und das Übertragen (390) des aktualisierten oralen Bildes an einen Zahnarzt oder Dentaltechniker.

3. Die Methode nach Anspruch 2, welche weiterhin umfasst: den Schritt der Speicherung (380) des aktualisierten oralen Bildes im Speicher.

4. Die Methode nach Anspruch 1, welche weiterhin umfasst: Die Aktualisierung (332) der Ausrichtung des Bildgebungsgeräts, wenn das Bildgebungsgerät eine Bewegung erkennt, welche einen vorbestimmten Schwellwert übersteigt.

5. Die Methode von Anspruch 1, wobei die Rückmeldung eine Audio, visuelle und/oder haptische Rückmeldung umfasst.

6. Die Methode von Anspruch 1, wobei das Bildgebungsgerät ein Smartphone ist, der Bildgeber eine Smartphone-Kamera.

7. Die Methode von Anspruch 1, wobei einer oder mehrere der Sensorparameter der Bildgebung durch die Bildeigenschaften an einem oder mehreren Punkten innerhalb eines erhaltenen oralen Bildes gesteuert oder bestimmt werden.

8. Die Methode von Anspruch 1, wobei das Bildqualitätsmerkmal eine mengenmäßige Bestimmung von Unschärfe, mengenmäßige Bestimmung von Glanzlicht und/oder dynamische Bereichserkennung umfasst.

9. Bildgebungsgerät (100) zum Erhalten eines oder mehrerer oraler Bilder, umfassend: einen Bildgeber (10), der konfiguriert ist, um ein oder mehrere Bilder (90) zu erhalten; eine Benutzerschnittstelle (60), die so konfiguriert ist, dass sie einem Benutzer des Bildgebungsgeräts Rückmeldung gibt; ein Kommunikationsmodul (50), das konfiguriert ist, um ein oder mehrere orale Bilder zu übertragen; einen Ausrichtungssensor (70), der dazu konfiguriert ist, eine Orientierung des Bildgebungsgeräts zu bestimmen; und
ein Steuergerät (30), konfiguriert zum: (i) Extrahieren eines oder mehrerer Merkmale aus dem erhaltenen oralen Bild, wobei das extrahierte eine Merkmal oder die mehreren Merkmale mindestens ein Bildqualitätsmerkmal und ein Objekterkennungsmerkmal umfassen; (ii) Bestimmen, ob das eine oder die mehreren extrahierten Merkmale
einen vorbestimmten Merkmalsschwellenwert erfüllen;
(iii) Berechnen, falls das eine oder die mehreren extrahierten Merkmale den vorbestimmten Merkmalsschwellenwert nicht erfüllen, einer physikalischen Anpassung des Bildgebungsgeräts, die erforderlich ist, um den vorbestimmten Merkmalsschwellenwert zu erfüllen, wobei die Berechnung der erforderlichen physikalischen Anpassung auf dem einen oder den mehreren extrahierten Merkmalen und der ermittelten Ausrichtung des Bildgebungsgeräts beruhen; und (iv) dem Benutzer über die Benutzerschnittstelle eine Rückmeldung bezüglich des erhaltenen oralen Bildes zu geben, wenn das eine oder die mehreren extrahierten Merkmale den vorbestimmten Merkmalsschwellenwert nicht erfüllen, wobei die Rückmeldung Anweisungen zum Vornehmen der berechneten physikalischen Einstellung des Bildgebungsgeräts umfasst, die erforderlich sind, um den vorbestimmten Merkmalsschwellenwert zu erfüllen.

10. Das Bildgebungssystem von Anspruch 9, wobei das Steuergerät außerdem konfiguriert ist, (i) ein oder mehrere Merkmale aus einem aktualisierten oralen Bild zu extrahieren, das erhalten wird, nachdem eine physische Anpassung durch den Benutzer vorgenommen wurde, wobei das eine oder die mehreren extrahierten Merkmale mindestens ein Bildqualitätsmerkmal und ein Objekterkennungsmerkmal umfassen; (ii) durch das Entscheidungsmodul zu bestimmen, dass das eine oder die mehreren extrahierten Merkmale den vorbestimmten Merkmalsschwellenwert erfüllen; und (iii) das Kommunikationsmodul anzuweisen, das aktualisierte orale Bild zu übertragen.

11. Das Bildgebungsgerät von Anspruch 10, wobei das Steuergerät außerdem konfiguriert ist, das aktualisierte orale Bild im Speicher zu speichern.

12. Die Methode von Anspruch 10, wobei die Rückmeldung eine Audio, visuelle und/oder haptische Rückmeldung umfasst.

13. Die Methode von Anspruch 10, wobei das Bildgebungsgerät ein Smartphone ist und der Bildgeber eine Smartphone-Kamera.

14. Ein Bildgebungssystem (200), konfiguriert, um eines oder mehrere orale Bilder von hoher Qualität zu erhalten, umfassend:
ein Bildgebungsgerät (100) umfassend einen Bildgeber (10), der konfiguriert ist, um ein oder mehrere Bilder (90) zu erhalten; ein Kommunikationsmodul (50b), das konfiguriert ist, um ein oder mehrere orale Bilder zu übertragen; und einen Ausrichtungssensor (70), der dazu konfiguriert ist, eine Orientierung des Bildgebungsgeräts
zu bestimmen; und eine Verarbeitungsvorrichtung (120), umfassend: ein Kommunikationsmodul (50b), das so konfiguriert ist, dass es ein oder mehrere orale Bilder vom Bildgebungsgerät empfängt; und eine Steuerung (30), die konfiguriert ist zum: (i) Extrahieren eines oder mehrerer Merkmale (90') aus dem erhaltenen oralen Bild, wobei das extrahierte eine oder die mehreren Merkmale mindestens ein Bildqualitätsmerkmal und ein Objekterkennungsmerkmal umfassen; (ii) zu bestimmen, ob das eine oder die mehreren extrahierten Merkmale einen vorbestimmten Merkmalsschwellenwert erfüllen;
(iii) Berechnen, falls das eine oder die mehreren extrahierten Merkmale den vorbestimmten Merkmalsschwellenwert nicht erfüllen, einer physikalischen Anpassung des Bildgebungsgeräts, die erforderlich ist, um den vorbestimmten Merkmalsschwellenwert zu erfüllen, wobei die Berechnung der erforderlichen physikalischen Anpassung auf dem einen oder den mehreren extrahierten Merkmalen und der ermittelte Ausrichtung des Bildgebungsgeräts beruhen; und (iv) dem Benutzer über eine Benutzerschnittstelle (60a, 60b) eine Rückmeldung bezüglich des erhaltenen oralen Bildes zu geben, wenn das eine oder die mehreren extrahierten Merkmale den vorbestimmten Merkmalsschwellenwert nicht erfüllen, wobei die Rückmeldung Anweisungen zum Durchführen der berechneten physikalischen Anpassung des Bildgebungsgeräts umfasst, die erforderlich ist, um den vorbestimmten Merkmalsschwellenwert zu erfüllen.

15. Die Methode von Anspruch 14, wobei die Rückmeldung eine Audio, visuelle und/oder haptische Rückmeldung umfasst.

## Revendications

1. Procédé (300) pour obtenir une ou plusieurs images orales en utilisant un dispositif d'imagerie (100), comprenant:
obtenir (320), en utilisant un imageur (10) du dispositif d'imagerie, une image orale (90);
déterminer (330), par un capteur (70) du dispositif d'imagerie, une orientation du dispositif d'imagerie (100) ;
extraire (340), par un contrôleur (30) du dispositif d'imagerie, une ou plusieurs caractéristiques (90') de l'image orale obtenue, où la ou les caractéristiques extraites comprennent au moins une caractéristique de qualité d'image et une caractéristique de reconnaissance d'objet;
déterminer (350), par le contrôleur, si la ou les caractéristiques extraites satisfont à un seuil de caractéristique prédéterminé;
calculer (360), par le contrôleur, si la ou les plusieurs caractéristiques extraites ne satisfont pas le seuil de caractéristique prédéterminé, un ajustement physique du dispositif d'imagerie requis pour satisfaire le seuil de caractéristique prédéterminé, dans lequel le calcul de l'ajustement physique requis est basé sur la ou les plusieurs caractéristiques extraites et l'orientation déterminée du dispositif d'imagerie; et
fournir (370), via une interface utilisateur (60) du dispositif d'imagerie, un retour d'information à un utilisateur concernant l'image orale obtenue si la ou
les caractéristiques extraites ne satisfont pas le seuil de caractéristique prédéterminé, dans lequel le retour d'information comprend des instructions pour mettre en oeuvre l'ajustement physique calculé du dispositif d'imagerie requis pour satisfaire le seuil de caractéristique prédéterminé.

2. Procédé selon la revendication 1, comprenant en outre les étapes consistant à:
obtenir (320), à l'aide de l'imageur du dispositif d'imagerie, une image orale mise à jour après la mise en œuvre d'un ajustement physique;
extraire (340), par le contrôleur, une ou plusieurs caractéristiques de l'image orale mise à jour, dans lequel la ou les caractéristiques extraites comprennent au moins une caractéristique de qualité d'image et une caractéristique de reconnaissance d'objet;
déterminer (350), par le contrôleur, que la ou les plusieurs caractéristiques extraites satisfont au seuil de caractéristique prédéterminé; et transmettre (390) l'image orale mise à jour à un professionnel dentaire.

3. Procédé selon la revendication 2, comprenant en outre l'étape consistant à stocker (380) l'image orale mise à jour dans la mémoire.

4. Procédé selon la revendication 1, comprenant en outre la mise à jour (332) de l'orientation du dispositif d'imagerie si le dispositif d'imagerie détecte un mouvement au-dessus d'un seuil prédéterminé.

5. Procédé selon la revendication 1, dans lequel le retour comprend un retour audio, visuel et/ou haptique.

6. Procédé selon la revendication 1, dans lequel le dispositif d'imagerie est un smartphone et l'imageur est une caméra de smartphone.

7. Procédé selon la revendication 1, dans lequel un ou plusieurs paramètres de capteur d'imagerie sont contrôlés ou déterminés par des propriétés d'image en un ou plusieurs points dans une image orale obtenue.

8. Procédé selon la revendication 1, dans lequel la caractéristique de qualité d'image comprend la quantification du flou, la quantification de la surbrillance spéculaire, et/ou la détection de la gamme dynamique.

9. Dispositif d'imagerie (100) pour obtenir une ou plusieurs images orales, comprenant:
un imageur (10) configuré pour obtenir une ou plusieurs images (90);
une interface utilisateur (60) configurée pour fournir un retour à un utilisateur du dispositif d'imagerie;
un module de communication (50) configuré pour transmettre une ou plusieurs images orales;
un capteur d'orientation (70) configuré pour déterminer une orientation du dispositif d'imagerie; et
un contrôleur (30) configuré pour: (i) extraire une ou plusieurs caractéristiques de l'image orale obtenue, où la ou les caractéristiques extraites comprennent au moins une caractéristique de qualité d'image et une caractéristique de reconnaissance d'objet; (ii) déterminer si la ou les caractéristiques extraites satisfont un seuil de caractéristique prédéterminé;
(iii) calculer, si la ou les caractéristiques extraites ne satisfont pas le seuil de caractéristique prédéterminé, un ajustement physique du dispositif d'imagerie requis pour satisfaire le seuil de caractéristique prédéterminé, dans lequel le calcul de l'ajustement physique requis est basé sur la ou les caractéristiques extraites et l'orientation déterminée du dispositif d'imagerie; et (iv) fournir, via l'interface utilisateur, un retour à l'utilisateur concernant l'image orale obtenue si la ou les caractéristiques extraites ne satisfont pas le seuil de caractéristique prédéterminé, dans lequel le retour comprend des instructions pour mettre en oeuvre l'ajustement physique calculé du dispositif d'imagerie nécessaire pour satisfaire le seuil de caractéristique prédéterminé.

10. Dispositif d'imagerie selon la revendication 9, dans lequel le contrôleur est en outre configuré pour:
(i) extraire une ou plusieurs caractéristiques d'une image orale mise à jour obtenue après qu'un ajustement physique est mis en oeuvre par l'utilisateur, dans lequel la ou les caractéristiques extraites comprennent au moins une caractéristique de qualité d'image et une caractéristique de reconnaissance d'objet; (ii) déterminer, par le module de décision, que la ou les caractéristiques extraites satisfont le seuil de caractéristique prédéterminé; et (iii) diriger le module de communication pour transmettre l'image orale mise à jour.

11. Dispositif d'imagerie selon la revendication 10, dans lequel le contrôleur est en outre configuré pour stocker l'image orale mise à jour dans la mémoire.

12. Dispositif d'imagerie selon la revendication 10, dans lequel le retour d'information comprend un retour d'information audio, visuel et/ou haptique.

13. Dispositif d'imagerie selon la revendication 10, dans lequel le dispositif d'imagerie est un smartphone et l'imageur est une caméra de smartphone.

14. Système d'imagerie (200) configuré pour obtenir une ou plusieurs images orales de haute qualité, comprenant:
un dispositif d'imagerie (100) comprenant un imageur (10) configuré pour obtenir une ou plusieurs images orales (90); un module de communication (50b) configuré pour transmettre une ou plusieurs images orales; et un capteur d'orientation (70) configuré pour déterminer une orientation du dispositif d'imagerie; et un dispositif de traitement (120) comprenant: un module de communication (50b) configuré pour recevoir une ou plusieurs images orales du dispositif d'imagerie; et un contrôleur (30) configuré pour: (i) extraire une ou plusieurs caractéristiques (90') de l'image orale obtenue, dans lequel la ou les caractéristiques extraites comprennent au moins une caractéristique de qualité d'image et une caractéristique de reconnaissance d'objet; (ii) déterminer si la ou les caractéristiques extraites satisfont un seuil de caractéristique prédéterminé ; (iii) calculer, si la ou les caractéristiques extraites ne satisfont pas le seuil de caractéristique prédéterminé, un ajustement physique du dispositif d'imagerie requis pour satisfaire le seuil de caractéristique prédéterminé, dans lequel le calcul de l'ajustement physique requis est basé sur la ou les caractéristiques extraites et l'orientation déterminée du dispositif d'imagerie; et (iv) fournir, par l'intermédiaire d'une interface utilisateur (60a, 60b), un retour à l'utilisateur concernant l'image orale obtenue si la ou les caractéristiques extraites ne satisfont pas le seuil de caractéristiques prédéterminé, dans lequel le retour comprend des instructions pour mettre en oeuvre l'ajustement physique calculé du dispositif d'imagerie nécessaire pour satisfaire le seuil de caractéristiques prédéterminé.

15. Le système d'imagerie selon la revendication 14, dans lequel le retour comprend un retour audio, visuel et/ou haptique.
